# EUROPEAN PATENT APPLICATION

(11) **EP 3 156 386 A1**
(43) Date of publication of application: **19.04.2017**
(21) Application number: 15806667.0
(22) Date of filing: 09.06.2015
(51) Int. Cl.: C07C 5/333, C07C 7/11, C07C 11/167

(54) **METHOD FOR PREPARING BUTADIENE THROUGH OXIDATIVE DEHYDROGENATION REACTION**

(30) Priority: 11.06.2014 KR 20140071050
(71) Applicant: LG Chem, Ltd., Yeongdeungpo-gu Seoul 150-721 (KR)
(72) Inventor: KIM, Mi Kyung, Daejeon 305-738 (KR); LEE, Jeong Seok, Daejeon 305-738 (KR); LEE, Jae Ik, Daejeon 305-738 (KR); KIM, Dae Hyeon, Daejeon 305-738 (KR); LEE, Jong Ku, Daejeon 305-738 (KR)
(74) Representative: Goddar, Heinz J.
(86) International application number: PCT/KR2015/005770
(87) International publication number: WO 2015/190801

(57) **Abstract**

The present invention relates to a method for producing butadiene through an oxidative dehydrogenation reaction, the method including steps of a) introducing a first stream which includes C4 fraction, steam, oxygen (O₂), and nitrogen (N₂) into a reactor which is filled with catalyst to perform oxidative dehydrogenation reaction; b) selectively absorbing butadiene, which is obtained from the reactor, into an absorption solvent, and separating and removing C4 mixture other than the dutadiene and light gas product; and c) recovering and purifying the butadiene. Through integration of the gas separating and purifying steps by using the single absorption solvent during production of butadiene, equipment costs and operating costs accruing from repeated introduction and removal of solvent are reduced, thereby ensuring economic competitiveness of the process.

## Description

### CROSS-REFERENCE TO RELATED APPLICATION

This application claims the benefit of Korean Patent Application No. 10-2014-0071050, filed on June 11, 2014, the entire contents of which are hereby incorporated by reference.

### TECHNICAL FIELD

The present invention relates to a method for producing butadiene through an oxidative dehydrogenation reaction.

### BACKGROUND ART

Butadiene is an important basic chemical, and is used as an intermediate in numerous petrochemical products such as synthetic rubbers and electronic materials. As one of the most important basic fractions in the current petrochemical market, the demand for butadiene and value thereof are steadily increasing.

Methods for producing butadiene include methods of extracting from C4 fraction through naphtha cracking, direct dehydrogenation of normal-butene (n-butene), oxidative dehydrogenation of n-butene, etc. Among such methods, a method for producing butadiene through hydrogenation reactions of butane or butene is performed in such a way that nitrogen, steam, etc., in addition to raw materials are introduced in order to reduce the danger of explosion, and also to prevent coking of catalyst and to remove reaction heat. According to the above reaction, along with the primary product which is butadiene, carbon monoxide, carbon dioxide, etc. which are secondary products are additionally produced.

C4 mixture containing butadiene is obtained by separating and removing light gas products from the reaction product, and is purified to thereby obtain high-purity butadiene. Meanwhile, a portion or all or the gas products which were separated and removed may be recycled.

### DISCLOSURE OF THE INVENTION

### TECHNICAL PROBLEM

Obtaining high-purity butadiene from a reaction product which is obtained from an oxidative dehydrogenation reaction of butane or butene requires undergoing a gas separation step, a purification step, etc., and undergoing solvent introduction and removal processes at least twice. Consequently, there is a limitation of increased equipment and operating costs due to repeated introduction and removal of solvent.

Thus, unlike typical gas separation and purification steps for recovering high-purity butadiene, a single absorption solvent was used to integrate the gas separation and purification steps such that the high-purity butadiene could be recovered through merely a single introduction and removal of the solvent.

### TECHNICAL SOLUTION

An embodiment for realizing an objective of the present invention provides a method for producing butadiene through an oxidative dehydrogenation reaction which includes a) a step for introducing a first stream which includes C4 fraction, steam, oxygen (O₂), and nitrogen (N₂) into a reactor which is filled with catalyst to perform an oxidative dehydrogenation reaction; b) a step for selectively absorbing butadiene into an absorption solvent from a reaction product which was obtained from the reactor, and separating and removing the C4 mixture other than the butadiene and light gas product; and c) a step for recovering and purifying the butadiene.

An exemplary embodiment may further include d) astep for recycling and reintroducing into the reactor a second stream which includes one or more selected from the group consisting of C4 mixture other than the butadiene; and nitrogen and carbon dioxide (CO₂) which are among the light gas product which was separated in the step b), and discharging a third stream which includes a purge to the outside of the system.

### ADVANTAGEOUS EFFECTS

According to the present invention, unlike a typical gas separation and purification for recovering high-purity butadiene, the gas separation and purification may be integrated into a single step, and thus equipment costs and operating costs accruing from repeated introduction and removal of solvent may be reduced.

Therefore, the economic competitiveness of the butadiene production process is improved.

### BRIEF DESCRIPTION OF THE DRAWINGS

FIG. 1 illustrates that a gas separation step and a purification step are integrated by using a single absorption solvent, when recovering butadiene which is produced through an oxidative dehydrogenation reaction according to an embodiment of the present invention.
FIG. 2 illustrates that a gas separation step and a purification step are separately performed by using respective solvents for the separation and purification steps, when recovering the butadiene which is produced through an oxidative dehydrogenation reaction according to a typical technique.

### MODE FOR CARRYING OUT THE INVENTION

Hereinafter, exemplary embodiments of the present invention are described with reference to the accompanying drawings. Although the present invention was described with reference to embodiments illustrated in the drawings, they are merely descriptions of embodiments, and thus the technical scope and essential features and functions of the present invention are not limited thereto. In particular, the term "light gas" which is used throughout the present specification, including the claims and abstract, shall be construed as indicating gaseous components including nitrogen, oxygen, water vapor, carbon monoxide, carbon dioxide, etc. which are among reaction products obtained through an oxidative dehydrogenation reaction. Moreover, the term "active component" shall be construed as indicating components such as nitrogen, oxygen, unreacted raw material, butadiene, etc., which are active in a reaction which produces butadiene.

A method for producing butadiene through an oxidative dehydrogenation reaction according to an embodiment of the present invention includes a) a step for introducing a first stream which includes C4 fraction, steam, oxygen (O₂), and nitrogen (N₂) into a reactor which is filled with catalyst to perform an oxidative dehydrogenation reaction; b) a step for selectively absorbing butadiene into an absorption solvent from a reactant which is obtained from the reactor, and separating and removing a C4 mixture other than the butadiene and a light gas product ; and c) a step for recovering and purifying the butadiene.

Moreover, according to an embodiment of the present invention, d) a step for recycling and reintroducing into the reactor a second stream which includes one or more selected from the group consisting of C4 mixture other than the butadiene; and nitrogen (N₂) and carbon dioxide (CO₂) which are among the light gas product which is separated in the step b), and discharging a third stream which includes a purge to the outside of the system, may be further included.

### Butadiene Production System

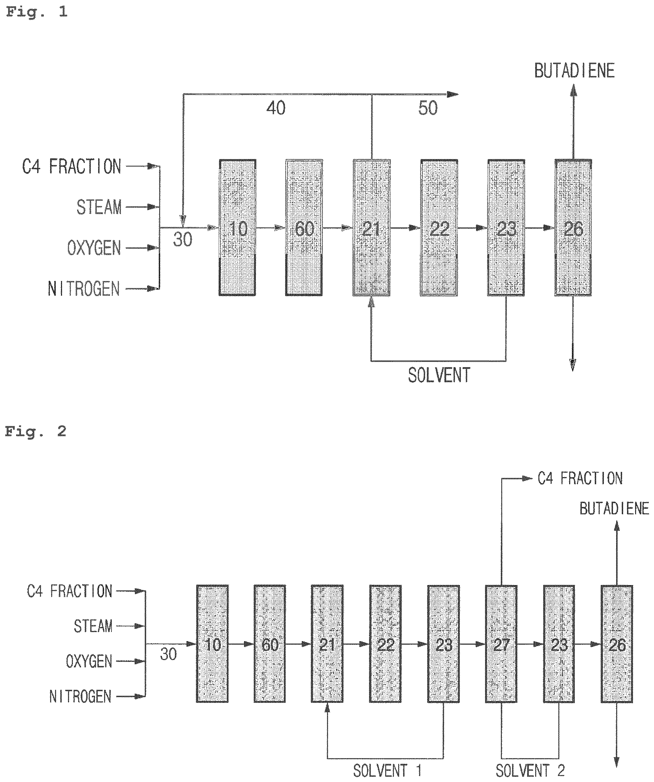

A butadiene production system for performing the above-described method for producing butadiene through the oxidative dehydrogenation reaction may include either individual pipelines for respectively introducing components of the first stream 30 including the C4 fraction, steam, oxygen (O₂), and nitrogen (N₂) into the reactor 10, or may include a plurality of individual pipelines which are branched out from a single pipeline directly connected to the reactor 10, and into which the components included in the first stream are individually introduced (see FIG. 1).

In addition, the reactor 10, which is connected to the pipeline and in which the oxidative dehydrogenation reaction occurs, is included. A mixing unit for mixing the components included in the first stream before the components flow into the reactor may be further included upstream of the reactor (see FIG. 1).

An absorption tower 21 and a gas stripper 22 are included which are provided for selectively absorbing the butadiene obtained from the reactor 10 into the absorption solvent, and for separating and removing the C4 fraction other than the butadiene and light gas product. A solvent recovery tower 23 may be included for obtaining only the butadiene from the absorption solvent (see FIG. 1).

A butadiene purification tower 26 for purifying the obtained butadiene to a high purity may be included (see FIG. 1).

Meanwhile, the butadiene production system of the present invention may further include an inert recycle line which allows, in the step d), the second stream 40, which includes one or more selected from the group consisting of C4 mixture; and nitrogen (N₂) and carbon dioxide (CO₂) which are among the light gas product which were separated in the step b), to be reintroduced into the reactor, and a discharge line for discharging the third stream 50 including the purge to the outside of the system (see FIG. 1).

In addition, a quenching unit provided with a quench tower for cooling the reaction product obtained from the reactor, a compressor for compression of the reaction product, and a dehydrator for removing moisture included in the reaction product, etc. may be further included between the reactor and gas separating unit.

### Butadiene Production Process

First, the oxidative dehydrogenation reaction is performed by introducing the first stream which includes the C4 fraction, steam, oxygen (O₂), and nitrogen (N₂) into the reactor (step a).

The C4 fraction may indicate C4 raffinate-1, 2, or 3 which remains after separation of useful compounds from the C4 mixture which was produced through naphtha cracking, or C4s which are obtained through ethylene dimerization. In an embodiment of the present invention, the C4 fraction may be one selected from the group consisting of n-butane, trans-2-butene, cis-2-butene, and 1-butene, or a mixture of at least two thereof.

In the oxidative dehydrogenation reaction, the steam and nitrogen (N₂) are diluting gases introduced in order to reduce the danger of explosion of the reactants, and also to prevent coking of the catalyst and to remove the reaction heat.

The oxygen (O₂) reacts as an oxidant with the C4 fraction and causes the dehydrogenation reaction to occur.

In an embodiment of the present invention, the first stream 30 may be a stream in which the C4 fraction, steam, oxygen (O₂), and nitrogen (N₂) flow into the reactor through respective individual pipelines.

In another embodiment of the present invention, the first stream 30 may be a stream in which the C4 fraction, steam, oxygen (O₂), and nitrogen (N₂), after passing through a plurality of individual pipelines which are branched out from a single pipeline that is directly connected to the reactor such that components included in the first stream are individually introduced, are mixed in the one pipeline or through the mixing unit which is located upstream of the reactor, and then introduced into the reactor.

In an embodiment of the present invention, the C4 fraction, steam, oxygen, and nitrogen which are included in the first stream may be introduced to the pipeline in a gaseous state, and the gas may also be introduced by preheating to a temperature which is advantageous for the oxidative dehydrogenation reaction.

In an embodiment of the present invention, the catalyst, which is charged into the reactor and allows the oxidative dehydrogenation reaction of the C4 fraction to thereby produce butadiene, is not particularly limited and may be, for example, a ferrite-based catalyst or a bismuth molybdate-based catalyst.

In an embodiment of the present invention, the catalyst may be the bismuth molybdate-based catalyst, and the bismuth molybdate-based catalyst may include one or more selected from the group consisting of bismuth, molybdenum, and cobalt. Moreover, the bismuth molybdate-based catalyst may also be a multicomponent bismuth molybdate-based catalyst. However, the type and amount of the reaction catalyst may differ according to the specific reaction conditions.

In an embodiment of the present invention, the reactor 10 in which the oxidative dehydrogenation reaction may be performed is not particularly limited. The reactor may be, for example, a tubular reactor, a tank reactor, or a fluidized bed reactor. In another example, the reactor may be a fixed bed reactor, and may also be a multitubular fixed bed reactor or a plate-type fixed bed reactor.

As described above, the oxidative dehydrogenation reaction is performed when the first stream 30 which includes the C4 fraction, steam, oxygen (O₂), and nitrogen (N₂) is introduced into the reactor 10 which is filled with the catalyst. The oxidative dehydrogenation reaction is an exothermic reaction, and the main reaction formula is Formula 1 or 2 which are below.

Formula 1 C₄H₈ + 1/2O₂ → C₄H₆ + H₂O

Formula 2 C₄H₁₀ + O₂ → C₄H₆ + 2H₂O

The butadiene is produced when hydrogen in butane or butene is removed through the oxidative dehydrogenation reaction. Side reactions, which are other than the main reactions such as those represented by Formula 1 or 2, may accompany the oxidative dehydrogenation reaction to thereby produce side reaction products such as carbon monoxide (CO), carbon dioxide (CO₂), etc. The side reaction products must be separated and discharged to the outside of the system in order to prevent a continuous build up in the process.

Meanwhile, the C4 mixture which includes the butadiene which is obtained from the reactor may further undergo a post-treatment process for obtaining the high-purity butadiene.

The post-treatment process may include one or more steps selected from among a quenching step in which the quench tower is used, a compressing step in which the compressor is used, and a dehydration step in which the dehydrator is used.

An embodiment of the present invention includes, as the post-treatment process, step for selectively absorbing the butadiene into the absorption solvent from the reaction product which is obtained from the reactor, and separating and removing the C4 fraction other than the butadiene and light gas product from the butadiene, and step for purification through the solvent recovery tower to recover solvent and the purification tower to purify the butadiene. In particular, unlike typical gas separation and purification steps, the gas separation and purification steps in the present invention are integrated into a single step by using a single absorption solvent.

### Quenching Step

In an embodiment of the present invention, the reaction product which is obtained from the reactor may undergo the quenching step.

The reaction product which is obtained from the reactor may be in a high temperature gaseous state, and thus need to be cooled before being supplied to the gas separating unit. Therefore, the reaction product may need to undergo the quenching step by being introduced to the quench tower.

The cooling method which is used in the quenching step is not particularly limited. For example, a cooling method in which the coolant directly contacts the reaction product may be used, and a cooling method in which the coolant indirectly contacts the reaction product may also be used.

### Dehydration Step

An embodiment of the present invention may further include the dehydration step in which moisture is removed from the reaction product obtained from the reactor.

Since, when moisture remains in the reaction product, there may be limitations in the subsequent solvent absorption, separation, and purification steps of equipment corrosion due to the moisture, or impurities build up in the solvent, the moisture must be removed.

A dehydration method in the dehydration step is not particularly limited. Moreover, the dehydration medium which is used in the dehydration step is not particularly limited and may be, for example, a desiccant (moisture absorbent) such as calcium oxide, calcium chloride, a molecular sieve, etc. Among such dehydration medium, a molecular sieve may be advantageous in terms of easy-recycling, easy-handling, etc.

### C4 Mixture and Light Gas Produce Separation and Removal Step

Unlike the typical art, in an embodiment of the present invention, among the reaction product obtained from the reactor, the butadiene is selectively absorbed by the absorption solvent, and the C4 mixture other than the butadiene and light gas productare separated and removed (step b).

Here, the reaction product may include at least about 90 mol% of the butadiene in the reaction product excluding the light gas product, and may specifically include the butadiene and C4 mixture at a molar ratio of about 93:7 to about 98:2.

In detail, when the reaction product obtained from the reactor makes counter-current contact with the absorption solvent in the absorption tower 21, only the butadiene is selectively absorbed by the absorption solvent, and the remaining C4 mixture other than the butadiene and light gas product exit through the top of the absorption tower.

The type of the absorption tower is not particularly limited and may be, for example, a packed tower, a wetted wall tower, a spray tower, a cyclone scrubber, a bubble tower, a bubble agitation tank, a tray tower (bubble cap tower, perforated plate tower), or a foam separation tower

Typically, it was required to undergo a process in which first the light gas product is removed by using the absorption solvent, and then, after separating only the butadiene by adding fresh solvent to the C4 mixture which includes the butadiene, the solvent is removed. Thus, a process in which the solvent is used at least twice was required. Consequently, there was a limitation of the equipment costs and operating costs being expensive.

However, according to the present invention, since butadiene may be selectively obtained by using and removing the solvent only once, the process is simple and the equipment costs, operating costs, etc. according to solvent usage are reduced.

The absorption solvent of the present invention must have butadiene selectivity. In an embodiment of the present invention, the absorption solvent may be a polar aprotic solvent.

In an embodiment of the present invention, the polar aprotic solvent may be one selected from the group consisting of dimethylformamide (DMF), methylpyrrolidone (NMP), acetonitrile (ACN), dimethylacetamide (DMA), and dimethyl sulfoxide (DMSO), or a mixture of at least two thereof.

Such the absorption solvent not only has a high solvent carrying capacity but, since selectivity to butadiene is high, may selectively absorb and dissolve only the butadiene from the reaction product.

The amount of the absorption solvent which is used is not particularly limited and, for example, may be modified according to the amount of the reaction product obtained from the reactor and the amount which is included in the third stream and thereby discharged to the outside of the system. Typically, economic competitiveness decreases as the amount of the absorption solvent which is used increases, and recovery efficiency of the butadiene decreases as the amount of the absorption solvent which is used decreases.

In an embodiment of the present invention, the C4 mixture other than the butadiene and light gas product, which was passed through the top of the absorption tower and discharged through piping, are divided into the second stream and the third stream.

The second stream may be a concentrated stream which includes one or more selected from the group consisting of nitrogen and carbon dioxide, and is recycled along an internal recycle line to be reintroduced into the reactor (step d of the present invention). Unreacted raw material, which are other than nitrogen (N₂) and carbon dioxide (CO₂), butadiene, etc. may be further included in the second stream, and the carbon dioxide which is included in the second stream may be reintroduced into the reactor through internal recycling to function in the reactor as a mild oxidant or as a diluting gas in the oxidative dehydrogenation reaction.

The third stream is discharged as a purge stream to the outside of the system through a separate discharge line from the second stream (step d of the present invention). The third stream may also further include nitrogen, carbon dioxide, unreacted raw material, butadiene, etc.

In an embodiment of the present invention, the absorption solvent is used for selectively absorbing butadiene, but may also dissolve portions of gasses such as nitrogen, carbon dioxide, etc. Thereby, the gas stripping step for removing gasses such as the nitrogen, carbon dioxide, etc. may be further performed, and such the gas stripping step may be performed in the gas stripper 22.

In the gas stripping step, the gas stripping method is not particularly limited, and may be a typical method which is used in the field.

### Butadiene Recovery _and_Purification Step

In an embodiment of the present invention, the absorption solvent which dissolved the butadiene is sent to the solvent recovery tower 23 for separating and recovering the absorption solvent.

In an embodiment of the present invention, the method for separating and recovering the solvent is not particularly limited and, for example, a distillative separation method may be used. According to the distillative separation method, after the absorption solvent which dissolved the butadiene is supplied to the solvent recovery tower 23, the distillative separation is performed by a reboiler and a condenser. By undergoing the distillative separation step, butadiene is extracted from near the top of the tower.

In the above step, the separated absorption solvent is extracted from the bottom of the solvent recovery tower, and the extracted absorption solvent may be reused by resupplying to the upstream process. Since the absorption solvent may include impurities, a portion of the absorption solvent may, prior to being recycled, be extracted and undergo a process in which the impurities are removed through a known purification method such as decantation, sedimentation, contact treatment with an absorption solvent or an ion exchange resin, etc.

The butadiene obtained from the top of the solvent recovery tower may be sent to the butadiene purification tower. In an embodiment of the present invention, since high boiling point and low boiling point components are removed as the butadiene which was sent to the purification tower passes through the purification tower, the butadiene is obtained as the high-purity butadiene.

In an embodiment of the present invention, the purity of the butadiene which may ultimately be obtained through the above series of steps is about 99.0% to about 99.9%.

### Examples

Hereinafter, the present invention will be described in more detail through examples. The examples are merely for illustrating embodiments of the present invention, and it will be obvious to those with ordinary skill in the art that the scope of the present invention is not limited by the examples.

### Example 1

According to the present invention, butadiene was produced in a reactor which was filled with a bismuth-molybdate based catalyst. Dimethylformamide (DMF) was used as an absorption solvent to selectively absorb the butadiene from among a reaction product which was obtained from the reactor, and only the butadiene was obtained through only a single solvent usage and removal process.

Specific reaction conditions and process flows were as shown in the following Table 1.

**[Table 1]**

| 1-butene (ton) | Solvent Usage (ton/BD ton) | Energy Usage (Gcal/BD ton) | | BD | |
|---|---|---|---|---|---|
| | | Gas Stripper | Solvent Recovery Tower | Flow Rate (ton) | Purity (wt%) |
| 1.12 | 14.77 | 0.50 | 0.82 | 1 | 99.7 |

### Comparative Example 1

Butadiene was produced through the same method as in Example 1, but a different solvent was used in each of gas separation and purification steps.

The reaction product which was obtained from the reactor entered an absorption tower which used toluene, and was thereby separated from light gas product. All of C4 mixture which includes butadiene was absorbed in the toluene. Next, the toluene which absorbed the C4 mixture which includes butadiene was sent to the solvent recovery tower, and thereby the toluene was recovered and the C4 mixture which includes butadiene was obtained.

The C4 mixture which includes butadiene was then supplied to a distillation tower, and dimethylformamide (DMF) introduced. In the distillation tower, the butadiene was selectively absorbed in the dimethylformamide, and the C4 which is other than the butadiene was discharged through the top of the distillation tower.

The dimethylformamide which includes the butadiene, after being supplied to the solvent recovery tower and then separated from the solvent and sent to a butadiene purification tower, went through a purification process and was thereby recovered as high-purity butadiene.

Thus, two of the solvent usage and recovery processes were needed in obtaining the high-purity butadiene.

Specific reaction conditions and process flows were as shown in the following Table 2.

**[Table 2]**

| 1-butene (ton) | Solvent Usage (ton/BD ton) | | Energy Usage (Gcal/BD ton) | | | | BD | |
|---|---|---|---|---|---|---|---|---|
| | Toluene | DMF | Gas Stripper | Solvent Recovery Tower | Distillation Tower | Solvent Recovery Tower | Flow Rate (ton) | Purity (wt%) |
| 1.12 | 10.7 | 9.81 | 0.26 | 0.42 | 0.42 | 0.36 | 1 | 99.7 |

By comparing results in Table 1 and Table 2, it was confirmed that a process according to an embodiment of the present invention is more efficient from the perspectives of energy usage and solvent usage.

## Claims

1. A method for producing butadiene through an oxidative dehydrogenation reaction, the method comprising:
a) a step for introducing a first stream which includes C4 fraction, steam, oxygen (O₂), and nitrogen (N₂) into a reactor which is filled with catalyst to perform an oxidative dehydrogenation reaction;
b) a step for selectively absorbing butadiene into an absorption solvent from a reactant which is obtained from the reactor, and separating and removing C4 mixture other than the butadiene and light gas product; and
c) a step for recovering and purifying the butadiene.

2. The method of claim 1, further comprising:
d) a step for recycling and reintroducing into the reactor a second stream which includes one or more selected from the group consisting of C4 mixture other than the butadiene; and nitrogen and carbon dioxide (CO₂) which are among the light gas product which is separated in the step b), and discharging a third stream which includes a purge to the outside of the system.

3. The method of claim 1, wherein the absorption solvent is a polar aprotic solvent.

4. The method of claim 1, wherein the absorption solvent is one selected from the group consisting of dimethylformamide (DMF), methylpyrrolidone (NMP), acetonitrile (ACN), dimethylacetamide (DMA), and dimethyl sulfoxide (DMSO), or a mixture of at least two thereof.

5. The method of claim 1, wherein the C4 fraction is a single compound or a mixture, which includes one or more selected from the group consisting of n-butane, trans-2-butene, cis-2-butene, and 1-butene.
